(19) Europäisches Patentamt
European Patent Office
Office européen des brevets

(11) **EP 0 547 210 B1**

(12) ## EUROPEAN PATENT SPECIFICATION

(45) Date of publication and mention
of the grant of the patent:
**03.01.1996 Bulletin 1996/01**

(21) Application number: **92915292.4**

(22) Date of filing: **03.07.1992**

(51) Int. Cl.$^6$: **A61M 1/16**, G05B 13/04

(86) International application number: **PCT/EP92/01498**

(87) International publication number: **WO 93/00938**
(21.01.1993 Gazette 1993/03)

(54) **METHOD AND MEANS FOR DIALYSIS**

VERFAHREN UND VORRICHTUNG ZUR DIALYSE

PROCEDE ET APPAREIL POUR DIALYSE

(84) Designated Contracting States:
**BE DE DK ES FR GB IT NL**

(30) Priority: **05.07.1991 IT TO910527**

(43) Date of publication of application:
**23.06.1993 Bulletin 1993/25**

(73) Proprietor: **HOSPAL LTD.**
**CH-4008 Basel (CH)**

(72) Inventors:
• **ROSSI, Marco**
**I-41036 Medola (IT)**
• **PAOLINI, Francesco**
**I-87100 Cosenza (IT)**

(56) References cited:
EP-A- 0 347 345         DE-A- 2 734 075
DE-A- 2 825 134         US-A- 4 718 891

• IEEE CONTROL SYSTEMS SOC. m. lau
:"proceedings of the 29th conference on
decision and control" 5 December 1990 , see
page 3162-3167, see fig 1
• 'Proceedings of the annual int. confer. of the ieee
engin. in medic. and biology soc. ' 4 November
1988 , G.HARRIS , NEW ORLEANS e. sarti : p.602-
604
• IEE PROCEEDINGS D. CONTROL THEORY &
APPLICATIONS. vol. 131, no. 4, June 1984,
STEVENAGE GB pages 117 - 124 D.WILLIAMS
'online adaptive control of a fermentation
process', see figures 1-2

EP 0 547 210 B1

## Description

This invention relates to a method of monitoring a dialysis unit and means for dialysis.

It is known that in order to avoid the adverse effects which frequently arise in patients subjected to haemodialysis treatment, in particular hypotension while dialysis is in progress, it is desirable to monitor the patient's blood volume measured from the percentage change in plasma volume (hereafter also indicated by the abbrevation CPV) in comparison with the start of the session. A parameter which can represent the patient's wellbeing satisfactorily is systolic arterial pressure, which is known to be correlated with the CPV.

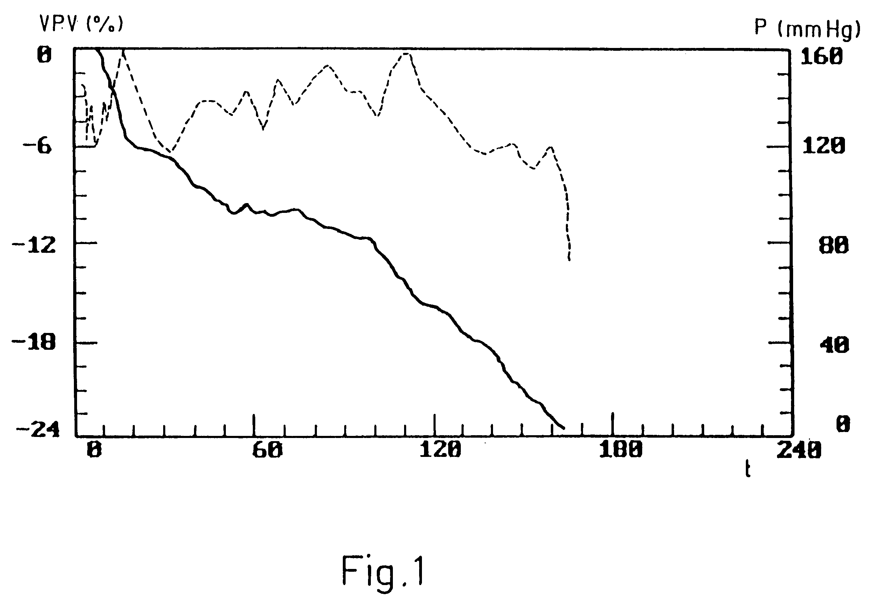

In this respect see for example Figure 1 which shows the percentage change in plasma volume (CPV, as a continuous line) and systolic arterial pressure (P, as a dashed line) during a dialysis session marked by the occurrence of severe hypotension at approximately time t = 170 min. As will be noted this is marked by an appreciable fall in CPV at the instant of collapse, in comparison with the start of the session (over 20%), and by a fast fall in this parameter (as will be seen from the strongly negative slope of the curve) during a time interval of approximately 1 hour prior to the hypotension, up to the moment of its occurrence.

Various systems for measuring changes in plasma volume which are applied in a variety of contexts are currently known. In the field of dialysis, equipment is also available which can vary certain machine parameters of clinical significance in the course of a session, such as for example the rate of weight loss and the osmolarity of the dialysing solution. These machines make it possible for the operator to define certain profiles before starting a dialysis session and then to administer treatment in such a way that the parameters in question adopt the values specified by the profile at various points in time. In all cases these parameters relate to the machine and as any further information is lacking the operator is unable to know the effect of any change in these on the patient, and in particular on his physiological parameters.

Systems which monitor some patient parameters are described for example in patent applications EP-A-29 793 and EP-A-89 003. In particular patent EP-A-29 793 in the name of Thomasset describes a system comprising a blood impedance measuring device and a device which infuses amounts of sodium chloride into the patient when the measured impedance value departs from a predetermined threshold. This system provides simple feedback of the on-off type which is hardly effective from the clinical point of view.

Patent EP-A-89 003 in the name of K. K. Toyota Chuo Kenkyusho instead describes a device based on a fundamental direct control system which causes the blood volume to follow a profile which is predetermined at the start of treatment on the basis of the patient's condition. In this known system the haematocrit is measured to obtain the changes in blood volume. However it has been shown that measurement of the haematocrit does not make it possible to determine changes in blood volume, as the hypothesis on which the mutual relationship between haematocrit and changes in blood volume, i.e. the hypothesis of constant total cell volume, is based, is rarely confirmed. In any event the proposed type of monitoring is poorly adapted for application to a whole population of patients or to different sessions with a given patient, or even to different times within a given session, as it does not take any account of individual reactions to treatment, which are not only generally different from patient to patient but can also vary in the same individual at different times.

Also this patent does not take into account the constraints to which manipulation of the machine parameters is subjected. For theses reasons the system described is poorly effective in situations where significant changes occur in the behaviour of the patient in the course of dialysis.

Patent document DE-A-2 825 134 discloses an automated hemodialysis method and a corresponding system which comprises a dialysis unit connectable to a patient, a CPU including a memory for storing desired values of a patient parameter, one or more blood analyzers for measuring the actual values of the patient parameters, and a control unit connected to the analyzer and the memory to receive actual values and desired values, wherein the CPU controls many of the machine parameters (for example speed of the pump, control unit, blood pump).

In such a system, individual reactions to treatment varying from patient to patient and in the same individual are not taken in account.

The document entitled "Parameter Set Estimation of Systems with Uncertain Nonparametric Dynamics and Disturbances" (proceedings of the 29th IEEE conference on decision and control, December 5-7 1990) generally discloses a method for parameter set estimation in order to design an adaptive control system for a non-specified plant. By additionally taking in account parameter set estimates derived from finite data records nonparametric uncertainty can be respected in controlling plants.

The object of this invention is to provide a method and means of dialysis which is capable of increasing the patient's wellbeing by reducing undesirable side effects to a minimum, and of achieving at least the same effectiveness as traditional treatment, achieving the cleansing objectives normally sought (e.g. monitoring weight loss and sodium removal).

This invention provides a dialysis system comprising a dialysis unit which is connected in use to a patient who is being subjected to dialysis treatment, a memory for storing desired values, which can vary in the course of time, of a patient parameter, at least one sensor for measuring the effective values of this patient parameter and a control section connected to the said memory and to the said sensor to receive the said actual and desired values of the said patient parameter, the said control section being capable of determining the value of at least one machine parameter supplied to the said dialysis unit to cause a said patient parameter to adopt the said desired values, characterised in that the said control section comprises an adaptive controller comprising estimator means capable of estimating the value of patient parameters correlated with the patient's response to dialysis treatment and monitoring means capable of determining the value of the said at least one machine parameter on the basis of the estimated value of the said patient parameters.

The invention also relates to a method of dialysis by means of a dialysis unit which is connected in use to a patient who is subjected to dialysis treatment comprising the stages of : storing desired values, which can change in the course of time, of a patient parameter in memory, measuring the actual values of the said patient parameter and monitoring the operation of the said dialysis unit by means of at least one machine parameter to cause the said patient parameter to adopt the said desired values, characterised in that the said stage of monitoring function incorporates monitoring of the adaptive type which includes estimation of the value of the patient parameters correlated with the patient's response to treatment and monitoring of the said machine parameter on the basis of the estimated value of the said patient parameters.

In practice this system can monitor a parameter which is extremely important to the wellbeing of the patient, such as the relative (percentage) change in plasma volume, via a feedback mechanism between the dialysis unit and the patient. In other words this system evaluates the patient's response moment by moment to stresses imposed by the dialysis unit and adjusts control on the basis of this knowledge. This feedback control is based on a mathematical model of the dialysis unit-patient system. Once the structure of this model has been defined (for example by means of a linear model described in the state memory), the model is completely individualised by values of its parameters which in general vary in the course of time (time variable model). These parameters describe the patient's response to dialysis treatment in a quantitative way, and a knowledge of their instantaneous values provides valuable information to the clinical operator.

For a better undestanding of this invention a preferred embodiment will now be described purely by way of a non-restrictive example with reference to the appended drawings in wich :

- Figure 1 shows the measured changes in the course of time of two parameters correlated with the wellbeing of a patient subjected to haemodialysis in the course of a session,

- Figure 2 shows a block diagram of the system according to this invention,

- Figure 3 shows a flow diagram corresponding to the algorithm used by the system in Figure 2 and,

- Figures 4 - 7 show the changes in time in the mean values and the standard deviations of the said two parameters in Figure 1, for a series of treatments of the traditional type and a series of treatments controlled according to the invention respectively, for a particular patient.

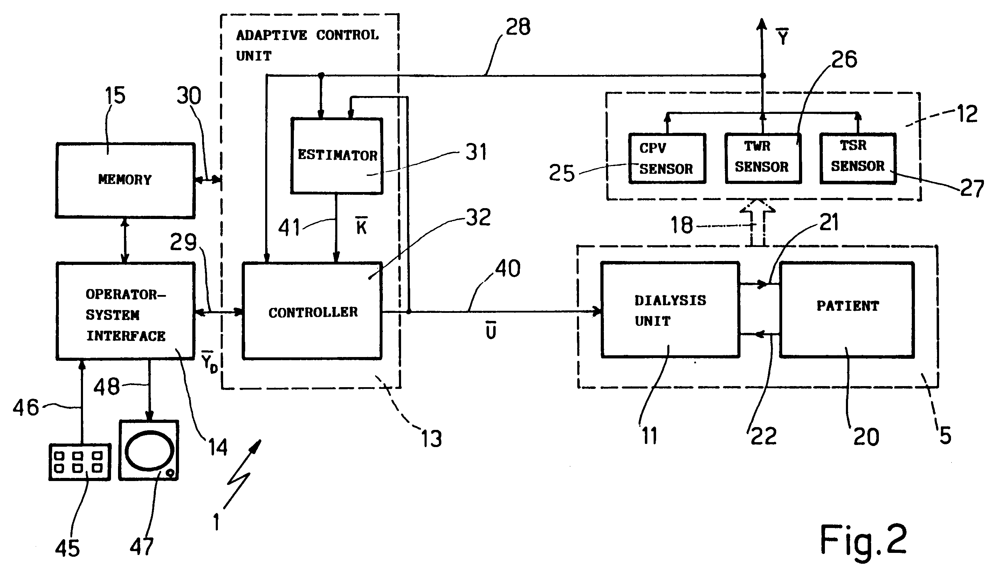

With reference to Figure 2, the dialysis system according to the invention is indicated as a whole by the numeral 1. System 1 comprises a dialysis unit 11 receiving as an input programmable machine parameters (vector $\overline{U}$), a group of sensors 12 for measuring the patient parameters which have to be monitored (vector $\overline{Y}$), a control unit 13 of the adaptive type which by acting on the machine parameter $\overline{U}$ which this provides to dialysis unit 11 causes the patient parameters $\overline{Y}$ to undergo desired changes (vectors $\overline{Y_D}$) which are predetermined by an operator through a system-operator interface 14 and stored in a memory 15.

System 1 as a whole operates in a discrete manner, i.e. the actual patient parameters $\overline{Y}$ and the desired patient parameters $\overline{Y_D}$ and the machine parameters $\overline{U}$ are monitored at predetermined time intervals and therefore define vectors $\overline{Y_i}$, $\overline{Y_{Di}}$, and $\overline{U_i}$ for each control cycle. This time interval between one cycle and the next, called the sampling interval $T_s$, is for example equal to 30 s.

In a known way dialysis unit 11, which is substantially of the traditional type, is connected to the patient's circulatory system, indicated diagrammatically by block 20 in Figure 2, via a pair of lines 21 and 22, which leave and enter the dialysis unit respectively. From the point of view of the adaptive control applied via control unit 13, dialysis unit 11 and patient 20 form a unit 5, and the connection between this and sensor group 12 is shown diagrammatically by arrow 18.

In the embodiments illustrated, the machine parameters provided as inputs to dialysis unit 11 from control unit 13 via line 40 are three in number and include the rate of weight loss, subsequently also indicated by RWL, the osmolarity of the dialysing solution, indicated by ODS, and the rate of infusion RIN.

As will be seen from Figure 2, sensor group 12 includes three sensors, specifically : a first sensor 25 to measure the percentage change in plasma volume CPV, a second sensor 26 to measure total weight loss TWL (difference in the patient's weight) at the instant in question, and a third sensor 27 to measure total sodium removal TSR up to the instant in question. These parameters CPV, TWL and TSR determine the patient parameters provided as inputs to control unit 13 via line 28. In detail second sensor 26 for calculating the total weight loss is of a known type and may be incorporated in dialysis unit 11. Third sensor 27 calculates total sodium removal, e.g. by measuring the sodium concentrations entering and leaving the dialysis unit and measuring the dialysing flow. First sensor 25 for measuring CPV consists of e.g. a suitable sensor which is located in the extracorporeal blood circulation line at the outlet from dialysis unit 11 and measures the infra red radiation absorption due to blood by means of optical devices (e.g. in the manner described in european patent application A-0467804 filed on 15.7.91 in the name of the same applicant), determines the haemoglobin (Hb) concentration from this absorption using a stored transfer characteristic (as described in the aforesaid european application), automatically determines a zero point at the start of the dialysis represented by a certain haemoglobin concentration value ($Hb_o$) and a null value for the percentage change in plasma volume, and determines the value of CPV from one instant to another on the basis of the following equation :

$$CPV = 100 \times (Hb_o/Hb - 1) \tag{1}$$

In fact, if PV is the absolute value of the plasma volume, Q is the quantity of haemoglobin and $PV_o$, $Q_o$ are the values of these parameters at the time zero, then :

$$Hb = Q/PV$$

$$Hb_o = Q_o/PV_o$$

$$CPV = 100 \times (PV - PV_o)/PV_o$$

$$= 100 \times [(Q/Q_o) \times (Hb_o/Hb) - 1]$$

On the assumption, which is in practice true while dialysis is in progress, that the amount of haemoglobin remains constant, that is $Q = Q_o$, equation (1) is obtained from the last line.

Control unit 13 is connected via a line 29 to system-operator interface 14 so it can read the desired values $\overline{Y_D}$ of the patient parameters, input the functional status (manual or automatic or session interruption), and input the sampling time $T_s$, the session time T, the final weight loss FWL, the final sodium removal FSR and all the machine parameters which are characteristic of conventional dialysis. Control unit 13 is also connected via a line 30 to memory 15 which exchanges the date necessary for their calculation and the results of the calculations. The two lines 29 and 30 are shown separately purely for the purpose of illustrating the method described here, which presupposes that the desired profiles are defined, while in practice these form a single connection.

In detail control unit 13 comprises an estimator 31 and a controller 32, both forming an adaptive controller and shown separately purely for the purposes of illustration, but in general represented in practice by a single component.

Estimator 31, which is based on a mathematical model describing the patient-dialysis unit system 5 as an isolated system with three inputs (machine parameters $\overline{U}$) and three outputs (patient parameters $\overline{Y}$), calculates the instantaneous values of the patient parameters $\overline{K}$ which are correlated with the patient's response to dialysis treatment at any given moment (i.e. the parameters of the mathematical model of the patient-dialysis unit system 5), as will be described in detail below.

The patient parameters obtained in this way are passed along a line 41 from estimator 31 to controller 32 whose function is to determine the present value of machine parameters $\overline{U}$ (RWL, ODS and RIN) on the basis of the state provided by system interface 14.

In particular, if the operator has requested conventional (manual) operation, controller 32 calculates constant values for the machine parameters, while if the operator has requested automatic (controlled) operation controller 32 calculates these values on the basis of a predetermined control relationship using the patient parameters $\overline{K}$ first calculated, as indicated in greater detail below. In any event controller 32 provides the instantaneous values of RWL, ODS, RIN as an output to dialysis unit 11 via line 40.

System-operator interface 14 is used for dialogue with the operator, for example via a keyboard 45, connected to interface 14 by a line 46 which enters the latter and a screen 47 which is connected to interface 14 by a line 47 which leaves the latter. Interface 14 is also connected to memory 15 for storing all relevant data for correct operation of the system. Interface 14 can therefore be used to input and store the desired profile $\overline{Y_D}$ for the patient parameters, provides control unit 13 with the necessary date and information at various times during the dialysis session, causes all information which is useful to the operator for evaluation of the session to be stored and displayed (instantaneous value of all variables in question) and can change the method of operation of the system (manual, automatic or end of session) at any time.

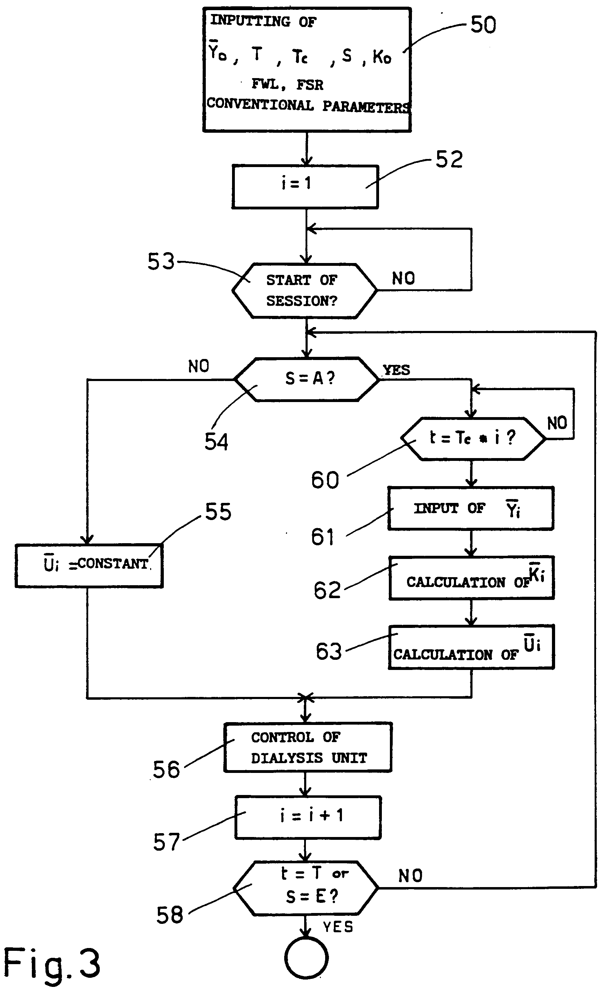

System 1 operates in the manner described below with reference to Figure 3 which describes the sequence of stages in a dialysis session.

In detail the session begins (block 50) with input of the desired profiles for the patient parameters by means of vector $\overline{Y_D}$, the session length T, the sampling time $T_s$, initial state of the system S, the initial values of the parameters of patient-dialysis unit system 5 and vector $\overline{K_o}$. Theses values may relate to an average patient defined in a conventional way, or better, they may result from an analysis of the average initial behaviour of the particular patient in previous sessions. The system also obtains the final weight loss FWL, the final sodium removal FSR and the parameters used in conventional dialysis (e.g. the temperature of the dialysing fluid). Subsequently (block 52) cycle counter i is initialised and the system awaits the session start command provided by the operator, e.g. by pressing a specific key on keyboard 45 (block 53). As soon as it receives the start command, control unit 13 begins the dialysis proper.

The dialysis treatment begins (block 54) with a check on the type of monitoring required, i.e. whether the setting is to manual (M) or automatic (A). This is because the operator can decide at any time to abandon automatic control and change over to manual control operating dialysis unit 11 in the conventional way and vice versa. If manual control is set (NO output from block 54) then block 55 comes into play which means controller 32 sets constant values for the machine parameters RWL, ODS and RIN, which are calculated taking past history into account. Then theses values which define a vector $\overline{U}_i$ are provided to dialysis unit 11 via line 40 (block 56). Then (block 57) controller 32 increments the cycle counter, and (block 58) checks that the session time has not reached termination (t=T) and that the operator has not requested an end to the session (S=E), and if this is not the case (NO output) it returns to block 54, checking the type of control required.

Vice versa, if automatic control is set (S=A), block 54 is followed by a block 60 in which a check is made to see whether the time interval specified between one cycle and the next has elapsed. If this is not the case (NO output from block 60) the system returns to block 54, but if the specified time has elapsed it passes from block 60 to block 61 relating to the stages of adaptive control in which estimator 31 and controller 32 calculate vector $\overline{U}_i$ for the current controlled values of the machine parameters.

In particular, in the generic cycle i, to begin with (block 61) estimator 31 receives vector $\overline{Y}_i$ which includes the values of patient parameters $RWL_i$, $TWL_i$, $TSR_i$ measured by sensors 25-27 at that moment, and then (block 62) estimator 31 calculates the existing value of the model parameters vector $\overline{K}_i$. To do this it uses a mathematical model of the patient-dialysis unit system 5, which is for example of the linear type characterised by parameters which are not known or which are variable in the course of time. One example of a mathematical model used, described in the state memory is as follows :

$$\overline{X} = A * \overline{X} + B * \overline{U}$$

$$\overline{Y} = C * \overline{X}$$

with $\overline{U}$ the vector for the inputs (machine parameters), $\overline{Y}$ the vector for the outputs (patient parameters), and

$$A = \begin{bmatrix} k1 & 0 & k2 \\ 0 & 0 & 0 \\ k3 & 0 & k4 \end{bmatrix} \qquad B = \begin{bmatrix} k5 & k6 & -k5 \\ 1 & 0 & -1 \\ k7 & k8 & b33 \end{bmatrix} \qquad C = 1$$

where b33 is a known term which depends on the type of solution used for infusion, k1-k8 are unknown parameters, which generally vary with time, and which constitute the vector $\overline{K}_i$ for the parameters calculated by the estimator for each cycle.

Estimator 31 is a least squares estimator which determines the value of parameters k1-k8 which minimise a cost function represented by the sum of the squares of the errors of the patient parameters, i.e. the squares of the differences between the actual values (measured by sensors 25-27) of the patient's parameters at various instants and the value predicted by the model for the actual sequence of inputs (machine parameters), for each cycle i using known algorithms.

After parameters k1 -k8 have been calculated, there then follows block 63 in which controller 32, starting from the desired values $\overline{Y}_D$ and the estimated patient parameters $\overline{K}$ determines the present values $\overline{U}_i$ of the machine parameters which will make it possible to obtain the desired profile $\overline{Y}_D$ for the patient parameters in accordance with a control relationship set by the controller itself. Example, controller 32 may consists of an LQR (Linear Quadratic Regulator) controller as described in the book by M. Tibaldi "Controlli automatic II", Pitagora Editrice, 1989. This controller is characterised in that it presupposes a linear model for the system under control (patient-dialysis unit system 5) and yields a cost function of the quadratic type which the control relationship minimises. From block 63 it then passes to blocks 56-58, which have already been described, which control dialysis unit 11, the counter increment and the check to establish whether the session has been ended. The stages described are then repeated until the end of the session (YES' output from block 58), after which treatment is interrupted.

The main advantage which can be obtained using the system according to this invention is due to the fact that this system effectively makes it possible to reduce the side effects which frequently arise during haemodialysis treatment without simultaneously reducing the effectiveness of the treatment. This is achieved by means of a feedback control which takes note of the behaviour of the individual patient at an individual instant during treatment, describing the patient-dialysis unit system using a model with parameters which vary in time so that the most significant patient parameters, such as the percentage change in plasma volume and therefore the patient's arterial pressure, can be controlled.

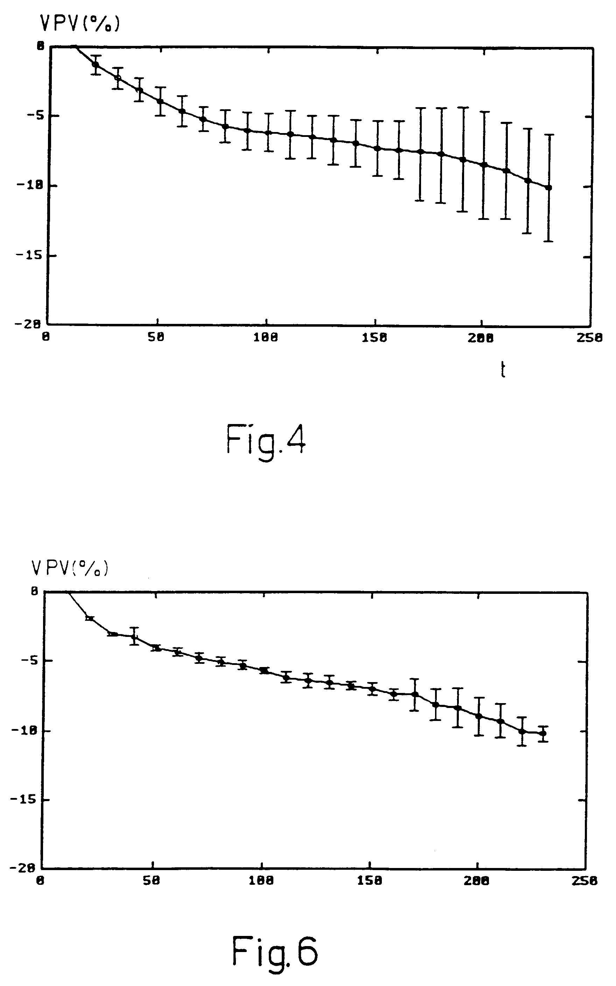

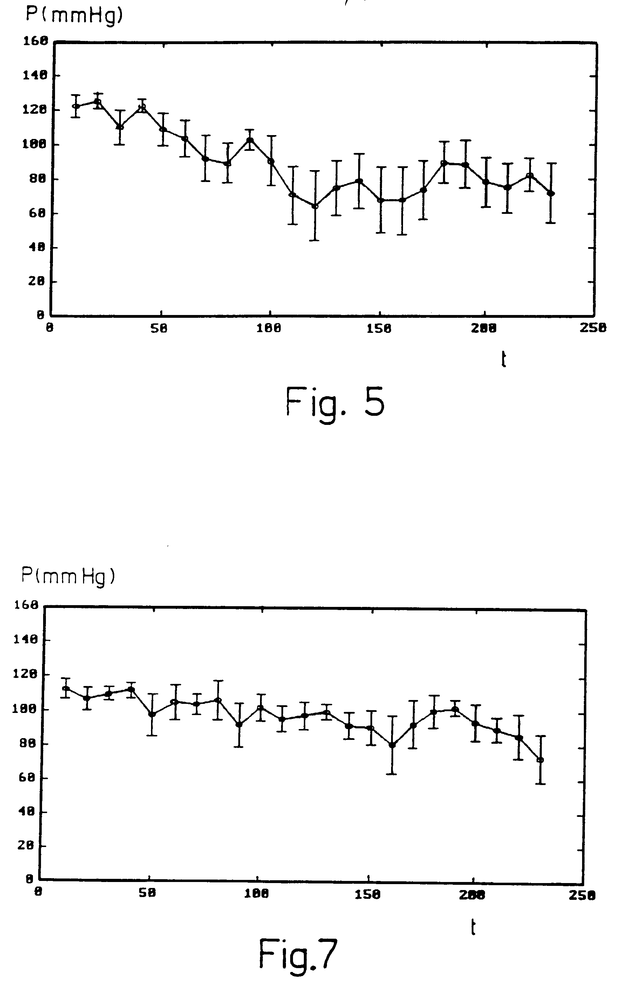

In this respect, as a demonstration of the effectiveness of the dialysis system according to this invention, reference should be made fo Figures 4 and 5, for example, which show the mean changes and the standard deviations for the CPV and systolic arterial pressure respectively during five conventional dialysis sessions involving the same critical patient. The appreciable scatter in the percentage change in plasma volume CPV resulting from the existence of sudden falls in that parameter and subsequent increases due to the infusion of physiological saline (one of the most common therapeutical measures following hypotension) is clear in Figure 4.

Conversely the fall in pressure leading to hypotension (pressure below 80 mm Hg) and the scatter of the pressure curve for the infusions carried out can clearly be seen in Figure 5. Figures 6 and 7 on the other hand show the change in the mean and standard deviation for the same parameters as in Figures 4 and 5 for the same patient when subjected to five automatic dialyses in accordance with the disclosures of this invention. These figures clearly show how the patient's wellbeing, i.e. increased stability of arterial pressure, is obtained through controlling the percentage change in plasma volume (reduced scatter with respect to the desired value along the line).

Another advantage of this system lies in the fact that the patients physiological response to dialysis treatment can be monitored with the parameters of the model of system 5 being quantified through the estimator. This information is of undoubted clinical interest merely for the supervision of a session, whether conventional or automatic.

Finally it is clear that modifications and variants may be made to the method and means described and illustrated here without thereby going beyond the scope of the protection provided by this invention. In particular it is emphasised that the model, the estimator and the controller may differ from those described. Also the system is suitable for controlling a variety of different clinical situations. In fact there is one category of patients in which the desired profile for changes in plasma volume can be obtained through suitable manipulation of the machine parameter RWL alone ; in this case the patient-dialysis unit system is reduced to a system with one input and one output, with a consequent reduction in the number of parameters which have to be determined and corresponding simplification of the estimator and the controller. For other categories of patients it is on the other hand necessary to make appropriate adjustments to the three machine parameters RWL, ODS and RIN, introducing constraints on such adjustments (e.g. the integral of the RWL must be equal to the patient's weight loss). In this case the system is one having three inputs, one output and constraints, and this has the advantage of being suitable for a larger number of patients and ensuring greater effectiveness for the treatment, particularly in terms of session times. Maximum effectiveness is however obtained by using the complete system with three inputs and three outputs described above, althrough at the expense of greater complexity.

## Claims

1. A dialysis system comprising a dialysis unit (11) which is connected when in use to a patient subjected to dialysis treatment, a memory (15) for storing desired values ($Y_D$), which vary in the course of time, of a patient parameter, at least one sensor (25) for measuring the actual values (Y) of the said patient parameter and a control unit (13) connected to the said memory (15) and to the said sensor (25) to receive the said actual values (Y) and desired values ($Y_D$) of the said patient parameter, the said control unit (13) being capable of determining the value (U) of at least one machine parameter passed to the said dialysis unit (11) to control the said patient parameter, characterised in that the said control unit (13) forms an adaptive controller comprising estimating means (31) capable of estimating the value of patient parameters (K) correlating with the patient's response to dialysis treatment and control means (32) for determining the value (U) of the said at least one machine parameter on the basis of the estimated values of the said patient parameters (K).

2. A dialysis system according to claim 1, characterised in that the said estimator means (31) comprise means (62) which are capable of estimating the values of the said patient parameters (K), at predetermined points in time, the latter representing the coefficients in a predetermined mathematical model which has as an input the said machine parameter (U) and as an output the said patient parameter (Y).

3. A dialysis system according to claim 2, characterised in that the said model is a linear model with parameters (K) which vary in the course of time.

4. A dialysis system according to claims 2 or 3, characterised in that the said estimating means (31) are capable of minimising an error function between the said actual value (Y) and a preset value of the said patient parameter obtained on the basis of the said mathematical model.

5. A dialysis system according to claim 4, characterised in that the said estimator means (31) consist of a least squares estimator acting on a sequence of errors.

6. A dialysis system according to one of the foregoing claims, characterised in that the said controller means (32) comprises means (63) capable of determining a controlled value (U) of the said machine parameter on the basis of the said patient parameters (K) and the said desired values ($Y_D$) of the said patient parameter.

**7.** A dialysis system according to claim 6, characterised in that the said controller means (32) consist of a quadratic linear controller.

**8.** A dialysis system according to one of the foregoing claims, characterised in that the said patient parameter consists of the relative change in plasma volume (CPV).

**9.** A dialysis system according to one of the foregoing claims characterised in that the said machine parameter consists of the rate of weight loss (RWL).

**10.** A dialysis system according to claim 9, characterised in that the said control unit (13) is capable of generating a further two machine parameters including the osmolarity of the dialysing solution (ODS) and the rate of infusion (RIN).

**11.** A dialysis system according to one of the foregoing claims, characterised in that the said at least one sensor (25) includes measurement means capable of measuring the haemoglobin concentration in blood.

**12.** A dialysis system according to claim 11, characterised in that the said measurement means incorporate an optical device for measuring the absorption of infra red radiations.

**13.** A dialysis system according to claim 11 or 12, characterised in that it comprises means capable of calculating the change in plasma volume CPV on the basis of the equation :

$$CPV = 100 \times (Hb_o/Hb - 1),$$

in which Hb is the haemoglobin concentration at the moment in question and $Hb_o$ is the haemoglobin concentration at the start of treatment.

**14.** A dialysis system according to one of claims 10 to 13, characterised in that it comprises a second sensor (26) capable of measuring actual values of total weight loss (TWL) and a third sensor (27) capable of measuring actual values of total sodium removal (TSR), the said second and third sensors being connected to the said control unit (13) for determining the values (U) of the said machine parameters.

**15.** A dialysis system according to claim 14, characterised in that the said memory (15) is capable of storing desired values $(Y_D)$ of the total weight loss and the total sodium removal which vary in the course of time.

**16.** A dialysis system according to one of the foregoing claims, characterised in that it comprises means (54) capable of switching between the automatic setting of machine parameters (RWL, ODS, RIN) and the manual setting of these at constant values.

**17.** A dialysis system according to one of the foregoing claims, characterised in that it comprises memory means (15) and a display (47) connected to the said control unit (13) for storing and displaying to an operator the instantaneous and mean values of the said machine (U) and patient (Y) parameters and the said patient parameters (K) which represent the patient's response to dialysis treatment.

**18.** A method of monitoring a dialysis unit (11) which is connected when in use to a patient subjected to dialysis treatment, comprising the stages of :

- storing in memory desired values $(Y_D)$ of a patient parameter, which vary in the course of time
- measuring actual values (Y) of the said patient parameter and,
- controlling the operation of the said dialysis unit (11) through at least one machine parameter (U) to cause the said patient parameter to adopt the said desired values,

characterised in that the said stage of the controlling of the operation is an adaptive control which includes the estimation (62) of patient parameters (K) correlating with the patient's response to treatment and the control (63) of the machine parameter (U) on the basis of estimated values of the said patient parameters.

**19.** A method according to claim 18, characterised in that the said stage of estimating the patient parameters (K) comprises the estimation at predetermined moments in time of the values of the said patient parameters (K) which represent the coefficients of a predetermined mathematical model which has as an input the said machine parameter (U) and as an output the said patient parameter (Y).

20. A method according to claim 19, characterised in that the said model is a linear model with parameters which vary in the course of time.

21. A method according to claims 19 or 20, caracterised in that the said stage of estimating the patient parameters (K) incorporates a stage of minimising an error function between the said actual value (Y) and a theoretical value of the said patient parameter obtained on the basis of the said mathematical model.

22. A method according to one of claims 19 to 21, characterised in that the said stage of estimating the patient parameters is based on a least squares estimator (32) acting on error sequences.

23. A method according to one of claims 18 to 22 characterised in that the said stage of adaptive control incorporates the stage of determining a controlled value of the said machine parameter (U) on the basis of the said estimated patient parameters (K) and the said desired value ($Y_D$) of the said patient parameter.

24. A method according to claim 23, characterised in that the said stage of adaptive control is based on a quadratic linear controller.

25. A method according to one of claims 18 to 24, characterised in that the said patient parameter consists of the relative change in plasma volume (CPV).

26. A method according to one of claims 18 to 25, characterised in that the said machine parameter consists of the rate of weight loss (RWL).

27. A method according to claim 26, characterised in that it also incorporates a stage of determining the values of a further two machine parameters comprising the osmolarity of the dialysing solution (ODS) and the rate of infusion (RIN).

28. A method according to claim 27, characterised in that it comprises the stages of : measuring actual values of the total weight loss (TWL) and measuring actual values of the total sodium removal (TSR), and in that the said model is a model with three inputs (U) and three outputs (Y).

29. A method according to one of claims 18 to 28, characterised in that the said stage of measuring the actual values (Y) of a patient parameter includes the stage of measuring the haemoglobin concentration in blood.

30. A method according to claim 29, characterised in that the said stage of measuring the haemoglobin concentration comprises an optical measurement of the absorption of infrared radiation.

31. A method according to one of claims 18 to 30, characterised in that it comprises the stages of storing in memory and displaying instantaneous and mean values of the said machine (U) and patient (Y) parameters and of the said patient parameters (K) which represent the patient's response to dialysis treatment.

**Patentansprüche**

1. Dialysesystem mit einem Dialysegerät (11), das bei Gebrauch an einen Patienten angeschlossen ist, der einer Dialysebehandlung unterzogen wird, einem Speicher (15) zur Speicherung von sich im Lauf der Zeit verändernden Sollwerten (YD) eines Patientenparameters, mindestens einem Fühler (25) zur Messung der Istwerte (Y) dieses Patientenparameters und einem mit dem Speicher (15) und dem Fühler (25) so verbundenen Regelgerät (13), daß es die Istwerte (Y) und Sollwerte (YD) des Patientenparameters empfängt, wobei das Regelgerät (13) imstande ist, den Wert (U) mindestens eines an das Dialysegerät (11) übermittelten Maschinenparameters zu ermitteln, um den Patientenparameter zu regeln, dadurch gekennzeichnet, daß das Regelgerät (13) einen adaptiven Regler mit einer der Abschätzung des Werts von mit dem Ansprechen des Patienten auf die Dialysebehandlung korrelierenden Patientenparametern (K) fähigen Abschätzeinrichtung (31) sowie einer Regeleinrichtung (32) zur Ermittlung des Werts (U) des mindestens einen Maschinenparameters aufgrund der Schätzwerte der Patientenparameter (K) bildet.

2. Dialysesystem nach Anspruch 1, dadurch gekennzeichnet, daß die Abschätzeinrichtung (31) eine Einrichtung (62) beinhaltet, die der Abschätzung der Werte der Patientenparameter (K) zu vorgegebenen Zeitpunkten fähig ist, die die Koeffizienten in einem vorgegebenen mathematischen Modell verkörpern, bei dem der Maschinenparameter (U) eine Eingabe und der Patientenparameter (Y) eine Ausgabe darstellt.

3. Dialysesystem nach Anspruch 2, dadurch gekennzeichnet, daß es sich bei dem Modell um ein lineares Modell mit sich im Lauf der Zeit verändernden Parametern (K) handelt.

4. Dialysesystem nach Ansprüchen 2 oder 3, daßdurch gekennzeichnet, daß die Abschätzeinrichtung (31) der Minimierung einer Fehlerfunktion zwischen dem Istwert (Y) und einem vorgegebenen Wert des Patienten-parameters, der aufgrund des mathematischen Modells erhalten worden ist, fähig ist.

5. Dialysesystem nach Anspruch 4, dadurch gekennzeichnet, daß die Schätz einrichtung (31) aus einem nach der Methode der kleinsten Quadrate mit einer Fehlerfolge arbeitenden Schätzer besteht.

6. Dialysesystem nach einem der vorhergehenden Ansprüche, dadurch gekennzeichnet, daß die Reglereinrichtung (32) eine der Ermittlung einer Regelgröße (U) des Maschinenparameters aufgrund der Patientparameter (K) und der Sollwerte (YD) des Patientenparameters fähige Einrichtung (63) beinhaltet.

7. Dialysesystem nach Anspruch 6, dadurch gekennzeichnet, daß es sich bei der Reglereinrichtung (32) um einen quadratisch-linearen Regler handelt.

8. Dialysesystem nach einem der vorhergehenden Ansprüche, dadurch gekennzeichnet, daß der Patientenparameter aus der relativen Plasmavolumenänderung (PVÄ) besteht.

9. Dialysesystem nach einem der vorhergehenden Ansprüche, dadurch gekennzeichnet, daß der Maschinenparameter aus der Gewichtsabnahmegeschwindigkeit (GAG) besteht.

10. Dialysesystem nach Anspruch 9, dadurch gekennzeichnet, daß das Regelgerät (13) der Generierung zweier weiterer Maschinenparameter einschließlich der Osmolarität der Dialyselösung (ODL) und der Infusionsgeschwindigkeit (ING) fähig ist.

11. Dialysesystem nach einem der vorhergehenden Ansprüche, dadurch gekennzeichnet, daß der mindestens eine Fühler (25) eine der Messung der Hämoglobinkonzentration im Blut fähige Meßeinrichtung beinhaltet.

12. Dialysesystem nach Anspruch 11, dadurch gekennzeichnet, daß die Meßeinrichtung eine optische Vorrichtung zur Messung der Absorption von Infrarotstrahlung enthält.

13. Dialysesystem nach Anspruch 11 oder 12, dadurch gekennzeichnet, daß es eine der Berechnung der Plasmavolumensänderung PVÄ aufgrund der Gleichung :

$$PVÄ = 100 \times (Hb0/Hb - 1),$$

wobei Hb die Hämoglobinkonzentration zum betreffenden Zeitpunkt und Hb0 die Hämoglobinkonzentration zu Beginn der Behandlung darstellt, fähige Einrichtung beinhaltet.

14. Dialysesystem nach einem der Ansprüche 10 bis 13, dadurch gekennzeichnet, daß es einen der Messung von Istwerten der Gesamtgewichtsabnahme (GGA) fähigen zweiten Fühler (26) sowie einen der Messung von Istwerten der Gesamtnatriumabnahme (GNA) fähigen dritten Fühler (27) beinhaltet, wobei der zweite und dritte Fühler an das Regelgerät (13) zur Ermittlung der Werte (U) der Maschinenparameter angeschlossen sind.

15. Dialysesystem nach Anspruch 14, dadurch gekennzeichnet, daß der Speicher (15) der Speicherung von Sollwerten (YD) der Gesamtgewichtsabnahme und der Gesamtnatriumabnahme, die sich im Lauf der Zeit ändern, fähig ist.

16. Dialysesystem nach einem der vorhergehenden Ansprüche, dadurch gekennzeichnet, daß es eine des Umschaltens zwischen der automatischen Einstellung von Maschinenparametern (GAG, ODL, ING) und der manuellen Einstellung derselben auf konstante Werte fähige Einrichtung (54) beinhaltet.

17. Dialysesystem nach einem der vorhergehenden Ansprüche, dadurch gekennzeichnet, daß es eine Speichereinrichtung (15) und eine Anzeige (47), an das Regelgerät (13) angeschlossen, beinhaltet, um die Istwerte und Mittelwerte der Maschinen-(U)- und Patienten-(Y)-Parameter sowie die das Ansprechen des Patienten auf die Dialysebehandlung verkörpernden Patientenparameter (K) zu speichern und einer Bedienungskraft anzuzeigen.

18. Verfahren zur öberwachung eines Dialysegeräts (11), das bei Gebrauch an einen Patienten angeschlossen ist, der einer Dialysebehandlung unterzogen wird, wobei das Verfahren die folgenden Etappen umfaßt:

- die Speicherung von Sollwerten (YD) eines sich im Lauf der Zeit ändernden Patientenparameters in einem Speicher,
- die Messung von Istwerten (Y) des Patientenparameters sowie
- die Regelung der Arbeitsweise des Dialysegeräts (11) über mindestens einen Maschinenparameter (U), um zu bewirken, daß der Patientenparameter die Sollwerte annimmt,

dadurch gekennzeichnet, daß es sich bei der Etappe der Regelung der Arbeitsweise um eine adaptive Regelung handelt, die die Abschätzung (62) von mit dem Ansprechen des Patienten auf die Behandlung korrelierenden Patientenparametern (K) sowie die Regelung (63) des Maschinenparameters (U) aufgrund von Schätzwerten der Patientenparameter beinhaltet.

19. Verfahren nach Anspruch 18, dadurch gekennzeichnet, daß die Etappe der Abschätzung der Patientenparameter (K) die Abschätzung zu vorgegebenen Zeitpunkten der Werte der Patientenparameter (K) beinhaltet, die die Koeffizienten in einem vorgegebenen mathematischen Modell verkörpern, bei dem der Maschinenparameter (U) eine Eingabe und der Patientenparameter (Y) eine Ausgabe darstellt.

20. Verfahren nach Anspruch 19, dadurch gekennzeichnet, daß es sich bei dem Modell um ein lineares Modell mit sich im Lauf der Zeit ändernden Parametern handelt.

21. Verfahren nach Ansprüchen 19 oder 20, dadurch gekennzeichnet, daß die Etappe der Abschätzung der Patientenparameter (K) eine Etappe der Minimierung einer Fehlerfunktion zwischen dem Istwert (Y) und einem aufgrund des mathematischen Modells erhaltenen theoretischen Wert des Patientenparameters beinhaltet.

22. Verfahren nach einem der Ansprüche 19 bis 21, dadurch gekennzeichnet, daß der Etappe der Abschätzung der Patientenparameter ein nach der Methode der kleinsten Quadrate mit einer Fehlerfolge arbeitender Schätzer (31) zugrunde liegt.

23. Verfahren nach einem der Ansprüche 18 bis 22, dadurch gekennzeichnet, daß die Etappe der adaptiven Regelung die Etappe der Ermittlung einer Regelgröße des Maschinenparameters (U) aufgrund der geschätzten Patientenparameter (K) und des Sollwerts (YD) des Patientenparameters beinhaltet.

24. Verfahren nach Anspruch 23, dadurch gekennzeichnet, daß der Etappe der adaptiven Regelung ein quadratisch-linearer Regler zugrunde liegt.

25. Verfahren nach einem der Ansprüche 18 bis 24, dadurch gekennzeichnet, daß der Patientenparameter aus der relativen Plasmavolumenänderung (PVÄ) besteht.

26. Verfahren nach einem der Ansprüche 18 bis 25, dadurch gekennzeichnet, daß der Maschinenparameter aus der Gewichtsabnahmegeschwindigkeit (GAG) besteht.

27. Verfahren nach Anspruch 26, dadurch gekennzeichnet, daß es auch eine Etappe der Ermittlung der Werte zweier weiterer Maschinenparameter beinhaltet, die Osmolarität der Dialyselösung (ODL) und die Infusionsgeschwindigkeit (ING) umfassen.

28. Verfahren nach Anspruch 27, dadurch gekennzeichnet, daß es die Etappen der Messung von Istwerten der Gesamtgewichtsabnahme (GGA) und der Messung von Istwerten der Gesamtnatriumabnahme (GNA) beinhaltet, und daß es sich bei dem Modell um ein Modell mit drei Eingaben (U) und drei Ausgaben (Y) handelt.

29. Verfahren nach einem der Ansprüche 18 bis 28, dadurch gekennzeichnet, daß die Etappe der Messung der Istwerte (Y) eines Patientenparameters die Etappe der Messung der Hämoglobinkonzentration im Blut beinhaltet.

**30.** Verfahren nach Anspruch 29, dadurch gekennzeichnet, daß die Etappe der Messung der Hämoglobinkonzentration eine optische Messung der Absorption von Infrarotstrahlung beinhaltet.

**31.** Verfahren nach einem der Ansprüche 18 bis 30, dadurch gekennzeichnet, daß es die Etappen der Speicherung in einem Speicher und der Anzeige von Istwerten und Mittelwerten der Maschinen-(U)- und Patienten-(Y)-Parameter sowie der das Ansprechen des Patienten auf die Dialysebehandlung verkörpernden Patientenparameter (K) beinhaltet.

**Revendications**

**1.** Système de dialyse comprenant une unité de dialyse (11) qui est connectée, lorsqu'elle est utilisée, à un patient soumis à un traitement de dialyse, une mémoire (15) pour mémoriser des valeurs souhaitées ($Y_D$), qui varient au cours du temps, d'un paramètre patient, au moins un capteur (25) pour mesurer les valeurs réelles (Y) du paramètre patient et une unité de commande (13) connectée à la mémoire (15) et au capteur (25) pour recevoir les valeurs réelles (Y) et les valeurs souhaitées ($Y_D$) du paramètre patient, l'unité de commande (13) étant capable de déterminer la valeur (U) d'au moins un paramètre machine fourni à l'unité de dialyse (11) pour commander le paramètre patient, caractérisé en ce que l'unité de commande (13) constitue un organe de commande adaptatif comprenant des moyens d'estimation (31) capables d'estimer la valeur de paramètres patient (K) en corrélation avec la réponse du patient au traitement de dialyse et des moyens de commande (32) pour déterminer la valeur (U) du paramètre machine en fonction des valeurs estimées des paramètres patient (K).

**2.** Système de dialyse selon la revendication 1, caractérisé en ce que les moyens d'estimation (31) comprennent des moyens (62) qui sont capables d'estimer les valeurs des paramètres patient (K), à des instants déterminés dans le temps, ces derniers représentant les coefficients d'un modèle mathématique prédéterminé ayant le paramètre machine (U) comme entrée et le paramètre patient (Y) comme sortie.

**3.** Système de dialyse selon la revendication 2, caractérisé en ce que le modèle est un modèle linéaire, les paramètres (K) variant au cours du temps.

**4.** Système de dialyse selon les revendications 2 ou 3 caractérisé en ce que les moyens d'estimation (31) sont capables de minimiser une fonction d'erreur entre la valeur réelle (Y) et une valeur prédéterminée du paramètre patient obtenue à partir du modèle mathématique.

**5.** Système de dialyse selon la revendication 4, caractérisé en ce que les moyens d'estimation (31) consistent un estimateur des moindres carrés agissant sur une séquence d'erreur.

**6.** Système de dialyse selon l'une des revendications précédentes caractérisé en ce que les moyens de commande (32) comprennent des moyens (63) capables de déterminer une valeur commandée (U) du paramètre machine en fonction des paramètres patients (K) et des valeurs souhaitées ($Y_D$) du paramètre patient.

**7.** Système de dialyse selon la revendication (6), caractérisé en ce que les moyens de commande (32) consistent en un organe de commande linéaire quadratique.

**8.** Système de dialyse selon une des revendications précédentes, caractérisé en ce que le paramètre patient est constitué par le changement relatif de volume du plasma (CPV).

**9.** Système de dialyse selon une des revendications précédentes, caractérisé en ce que le paramètre machine est constitué par le débit de perte de poids (RWL).

**10.** Système de dialyse selon la revendication 9, caractérisé en ce que l'unité de commande (13) est capable d'engendrer deux autres paramètres machine incluant l'osmolarité de la solution de dialyse (ODS) et le débit d'infusion (RIN).

**11.** Système de dialyse selon une des revendications précédentes, caractérisé en ce que le capteur (25) comprend des moyens de mesure capable de mesurer la concentration en hémoglobine du sang.

**12.** Système de dialyse selon la revendication 11, caractérisé en ce que les moyens de mesure comprennent un dispositif optique pour mesurer l'absorption des radiations infrarouges.

**13.** Système de dialyse selon la revendication 11 ou 12, caractérisé en ce qu'il comprend des moyens capables de calculer la variation du volume du plasma CPV à partir de l'équation :

$$CPV = 100 \times (Hb_o/Hb - 1)$$

dans laquelle Hb est la concentration en hémoglobine à un moment déterminé et $Hb_o$ est la concentration en hémoglobine au début du traitement.

**14.** Système de dialyse selon une des revendications 10 à 13, caractérisé en ce qu'il comporte un deuxième capteur (26) capable de mesurer les valeurs réelles de la perte de poids totale (TWL) et un troisième capteur (27) capable de mesurer les valeurs réelles de la déplétion totale du sodium (TSR), le second et le troisième capteurs étant connectés à l'unité de commande (13) pour déterminer les valeurs (U) des paramètres machine.

**15.** Système de dialyse selon la revendication 14, caractérisé en ce que la mémoire (15) est capable de stocker les valeurs désirées ($Y_D$) de la perte de poids totale et de la déplétion totale du sodium, qui varient au cours du temps.

**16.** Système de dialyse selon l'une des revendications précédentes, caractérisé en ce qu'il comprend des moyens (54) capables de commuter entre le réglage automatique des paramètres machine (RWL, ODS, RIN) et le réglage manuel de ces paramètres à des valeurs constantes.

**17.** Système de dialyse selon une des revendications précédentes, caractérisé en ce qu'il comporte des moyens de mémorisation (15) et des moyens d'affichage (47) connecté à l'unité de commande (13) pour mémoriser et afficher pour un opérateur les valeurs instantanées et les valeurs moyennes des paramètres machine (U) et des paramètres patient (Y) et les paramètres patient (K) qui représentent la réponse du patient au traitement de dialyse.

**18.** Procédé pour contrôler une unité de dialyse (11) qui est connectée, lorsqu'elle est utilisée, à un patient soumis à un traitement de dialyse, comprenant les étapes de :

- stocker dans une mémoire des valeurs souhaitées ($Y_D$) d'un paramètre patient, qui varie au cours du temps,
- mesurer les valeurs réelles (Y) du paramètre patient et,
- commander le fonctionnement de l'unité de dialyse (11) au moyen d'au moins un paramètre machine (U) pour faire que la paramètre patient adopte les valeurs souhaitées,

caractérisé en ce que l'étape de commander le fonctionnement est une commande adaptative qui inclut l'estimation (62) de paramètres patient (K) en corrélation avec la réponse du patient au traitement ainsi que la commande (63) du paramètre machine (U) à partir des valeurs estimées des paramètres patient.

**19.** Procédé selon la revendication 18, caractérisé en ce que l'étape d'estimer les paramètres patient (K) comprend l'estimation à des intants prédéterminés des valeurs des paramètres patient (K), qui représentent les coefficients d'un modèle mathématique prédéterminé ayant pour entrée le paramètre machine (U) et pour sortie le paramètre patient (Y).

**20.** Procédé selon la revendication 19, caractérisé en ce que le modèle est un modèle linéaire avec des paramètres qui varient dans le cours du temps.

**21.** Procédé selon les revendications 19 ou 20, caractérisé en ce que l'étape d'estimer les paramètres patient (K) comporte l'étape de minimiser une fonction d'erreur entre la valeur réelle (Y) et une valeur théorique du paramètre patient obtenue à partir du modèle mathématique.

**22.** Procédé selon une des revendications 19 à 21, caractérisé en ce que l'étape d'estimer les paramètres patient est fondée sur un estimateur de moindres carrés (32) agissant sur des séquences d'erreur.

**23.** Procédé selon une des revendications 18 à 22, caractérisé en ce que l'étape de commande adaptative comprend l'étape de détermination d'une valeur commandée du paramètre machine (U) à partir des paramètres patient (K) estimés et de la valeur souhaitée ($Y_D$) du paramètre patient.

**24.** Procédé selon la revendication 23, caractérisé en ce que l'étape de commande adaptative est fondée sur une commande linéaire quadratique.

**25.** Procédé selon une des revendications 18 à 24, caractérisé en ce que le paramètre patient est constitué par la variation relative de volume du plasma (CPV).

**26.** Procédé selon une des revendications 18 à 25, caractérisé en ce que le paramètre machine est constitué par le débit de perte de poids (RWL).

**27.** Procédé selon la revendication 26, caractérisé en ce qu'il comprend aussi une étape de détermination des valeurs de deux autres paramètres machine comprenant l'osmolarité de la solution de dialyse (ODS) et le débit d'infusion (RIN).

**28.** Procédé selon la revendication 27, caractérisé en ce qu'il comprend les étapes de : mesurer les valeurs réelles de la perte de poids totale (TWL) et mesurer les valeurs réelles de la déplétion totale du sodium (TSR), et en ce que le modèle est un modèle à trois entrées (U) et trois sorties (Y).

**29.** Procédé selon une des revendications 18 à 28, caractérisé en ce que l'étape de mesurer les valeurs réelles (Y) d'un paramètre patient comprend l'étape de mesurer la concentration en hémoglobine du sang.

**30.** Procédé selon la revendication 29, caractérisé en ce que l'étape de mesurer la concentration en hémoglobine comprend une mesure optique de l'absorption du rayonnement infrarouge.

**31.** Procédé selon une des revendications 18 à 30, caractérisé en ce qu'il comprend les étapes de stocker dans une mémoire et d'afficher des valeurs instantanées et moyennes des paramètres machine (U) et des paramètres patient (Y) et des paramètres patient (K) qui représentent la réponse du patient au traitement de dialyse.

Fig.1

Fig.2

EP 0 547 210 B1

INPUTTING OF
$\bar{Y}_0$ , $T$ , $T_c$ , $S$ , $K_0$
FWL, FSR
CONVENTIONAL PARAMETERS — 50

$i = 1$ — 52

53 — START OF SESSION?　　NO

NO　　$S = A$ ?　　YES

54

$t = T_c * i$ ?　　NO

60

61 — INPUT OF $\bar{Y}_i$

55 — $\bar{U}_i =$ CONSTANT

62 — CALCULATION OF $\bar{K}_i$

63 — CALCULATION OF $\bar{U}_i$

CONTROL OF DIALYSIS UNIT
56

$i = i + 1$
57

58 — $t = T$ or $S = E$ ?　　NO

YES

# Fig.3

Fig.4

Fig.6

P(mmHg)

Fig. 5

P(mm Hg)

Fig.7